# EUROPEAN PATENT SPECIFICATION

(11) **EP 2 449 983 B1**
(45) Date of publication and mention of the grant of the patent: **01.01.2014**
(21) Application number: 10190060.3
(22) Date of filing: 04.11.2010
(51) Int. Cl.: A61B 17/08, A61B 17/122, A61B 17/128

(54) **Surgical clip**
Chirurgische Klammer
Clip chirurgical

(43) Date of publication of application: 09.05.2012
(73) Proprietor: Ovesco Endoscopy AG, 72074 Tübingen (DE)
(72) Inventor: Baur, Franziska, 73265 Dettingen unter Teck (DE); Ho, Chi-Nghia, 70191 Stuttgart (DE); Schurr, Marc O., 72072 Tübingen (DE); Anhöck, Gunnar, 72762 Reutlingen (DE); Gottwald, Thomas, 82431 Kochel am See (DE)
(74) Representative: Winter, Brandl, Fürniss, Hübner, Röss, Kaiser, Polte - Partnerschaft

(56) References cited:
- EP-A1- 1 721 587
- WO-A2-02/45593
- US-A1- 2005 234 483
- US-B2- 6 849 078

## Description

The present invention relates to a surgical or tissue clip for minimal-invasive lesion treatment of a patient's tissue, for instance a stomach wall, a colon or the like.

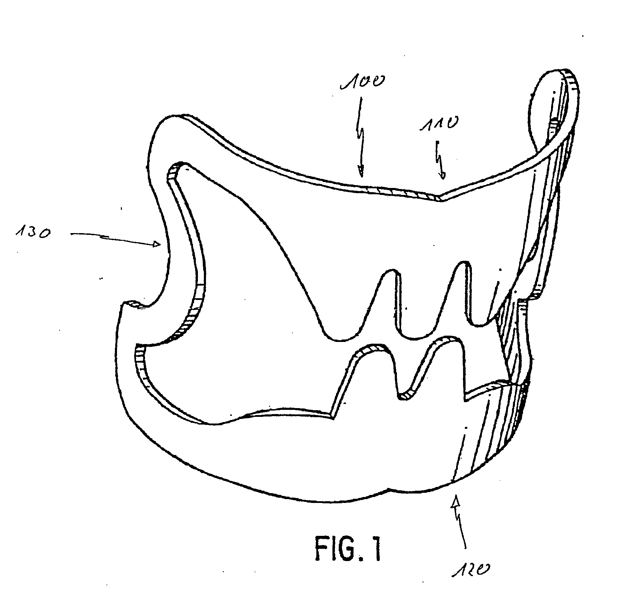

From the prior art, for instance according to US 6, 849,078 B2, which discloses a surgical clip according to the preamble of claim 1, of the present inventor, a tissue clip for closing a tissue cut of this kind is generally known as regards this basic construction. For a better comprehension, this clip is hereinafter described in more detail with reference to figure 1.

Accordingly, such a clip 100 consists of a mouth-like clamping means having two toothed jaws 110, 120 which can be opened and shut via two lateral hinges 130 or flexible moldings (flexible connection means like metal bands, strips, strings or the like). The hinges 130 or flexible moldings are preferably formed of spring-elastic straps which when opening the jaws 110, 120 store spring energy which results in snapping the jaws 110, 120 at a predetermined clamping force when the jaws 110, 120 are released, i.e. when the hinges 130 or the flexible moldings are actuated.

In detail, each clip 100 is punched or lasered in one piece out of a spring steel sheet by working a ring having a partially different ring width out of the spring steel sheet. Here, the term ring width means the dimension (extension) of the spring steel sheet in radial direction of the ring such that the ring-shaped spring steel sheet gets partially very small at at least two diametrical located portions and larger at portions there between.

The jaws 110, 120 will then be formed by arching the ring portions having the larger ring width in a curved / cambered shape over the originally flat side thereof, whereas the two ring portions having the narrow ring width are additionally twisted (change of their radial extension) about their longitudinal axis by approx. 180° in order to form the hinges. In other words, the radial direction of the hinge portions is substantially opposite to the radial direction of the jaw portions of the closed ring.

This special shaping of the preferably lasered spring steel sheet creates the shape of a type of a shark mouth having two rows of teeth moving toward each other which are formed by laser cutting the ring portions having a large ring width.

The general function of the afore-described medical tissue clip 100 can be described as follows:
In general, an endoscopic implantation of a medical device in total constitutes the most tolerable process for a patient. In this case the medical device must be fixed from the inside of a hollow organ to the latter. For this purpose, a number (at least one) of the afore-defined tissue cleats, clips or anchors are inserted into the hollow organ preferably by means of an endoscope or a similar feeding means and are positioned at predetermined points / positions at the inner side of the organ. To this end, the respective clip or anchor is brought near the organ tissue and the biasing springs / the elastic hinges are released for snapping of the clip or clamping of the anchor. The latter then holds or clamps a tissue fold between its jaws / teeth or needles cut / pierce into the tissue and preferably penetrate the same.

The endoscope or similar feed means not shown in detail in figure 1 usually is equipped with an endoscope head or an endoscope cap which includes, apart from the functions generally required for an endoscope such as lighting, optical system and rinsing means, if necessary, in addition a holding and withdrawing means for the tissue clip. It is referred to the fact in this context that in this entire application also a simple inserting aid without separate lighting and optical system as well as rinsing function can be understood by an endoscope.

The holding and withdrawing means substantially consists of an expanding sleeve as well as a slide operable manually or by remote control which is moveable in the longitudinal direction of the endoscope. The expanding sleeve is designed such that the already opened tissue clip can be placed onto the sleeve so that the clip can be prevented from slipping backward wile being inserted into the hollow organ, For this purpose, the slide is positioned axially behind the clip (seen in the forward direction of the endoscope) and serves as an axial stop for the clip.

As soon as the clip is to be positioned at a particular site, the slide is moved axially forward and in so doing strips off the clip over the expanding sleeve. The clip is actuated, i.e. the biasing mechanism within the clip described before by way of figure 1 is released when it is stripped off the expanding sleeve and the jaws of the tissue clip snap to close while clamping the tissue provided there between.

Besides, reference WO 02/45593 A2 discloses another surgical clip of this kind having a generally annular-shaped flat body defining a plane and a plurality of tissue grasping teeth extending from the annular-shaped body substantially transversely with respect to the plane. The tissue grasping teeth are biased towards a substantially planar configuration lying in the plane.

Accordingly, in a top view a symmetrical configuration is defined comprising an outer closed ring structure from which a plurality of teeth extend into the radial inward direction.

In view of the afore-described prior art it is an object of the present invention to provide a tissue clip of this kind having an increased functionality. One aim of the present invention is to enhance its tissue cramping abilities without increasing the tissue damaging risk because of the closing forces generated by the elastically deformed hinge portions.

This object is solved by a tissue clip having the technical features in accordance with the patent claim 1. Further advantageous configurations of the embodiment are subject matter of the sub claims.

Accordingly, the inventions provides a surgical clip, tissue clip or anchor preferably deliverable at a site within a patient's body by an endoscopic device. Said clip or anchor is formed as a closed ring made of an elastic metal sheet or another sheet-like material having elastic character. Furthermore, the ring-shaped clip is divided into a plurality of tissue grasping portions each formed with at least one tooth or a row of teeth extending substantially radial in the direction inside the ring and a plurality of elastically deformable hinge portions located between two neighbouring grasping portions in circumferential direction, respectively. The hinge portions of the ring-shaped clip have a curvature (orientation) extending in the direction inside the ring which means that the radius of each hinge portion is orientated opposite to the basis radius of the ring-shaped clip.

According to the invention, at least one of said grasping portions and said hinge portions are provided with an additional tooth or an additional row of teeth, extending in the direction to the adjacent other one of said hinge portions and grasping portions. In other words the grasping portions and/or the hinge portions comprise an additional tooth or row of teeth preferably on its both circumferential end portions, respectively which tooth or teeth extend radial inside the ring-shaped clip and are inclined (in superimposed manner) in the direction to the adjacent hinge portions and/or grasping portions.

Because the hinge portions have a curvature the radius thereof extending inside the ring-shaped clip, the circumference of each hinge portion and the circumferential end of each adjacent grasping portion are at least partially facing each other forming a gap there between. Furthermore, because an additional tooth or row of teeth is arranged in that gaps, additional grasping areas besides the grasping portions are created increasing the clamping capability of the clip or anchor.

Hereinafter the invention will be explained in more detail by way of preferred embodiments with reference to the accompanying drawings, in which
figure 1 shows a tissue clip according to the state of the art,
figures 2 to 4 show a tissue clip according to a first preferred embodiment of the invention from different side views and
figures 5 to 7 show a tissue clip according to a second preferred embodiment of the invention also from different side views.

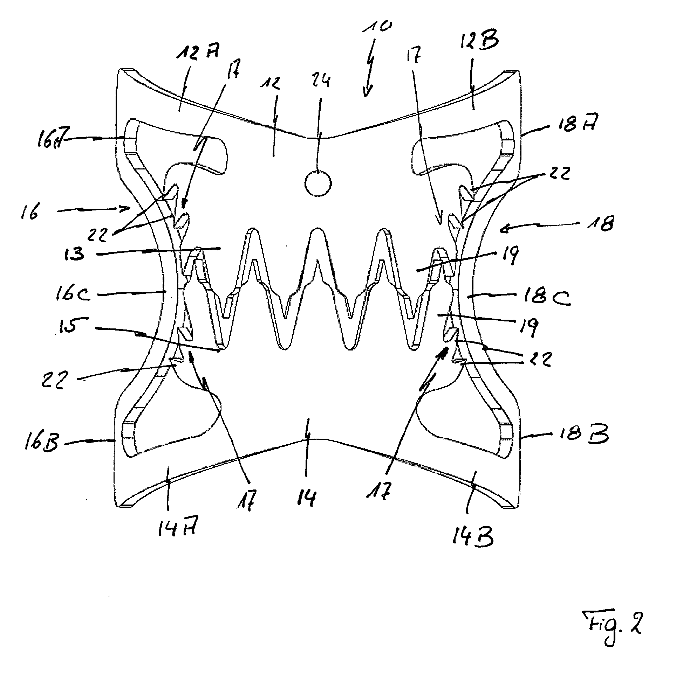

The figures 2 to 4 illustrate a first embodiment for a surgical clip according to the present invention, that may be delivered to a site within a patient's body by an endoscopic device as known for example from US 6,428 548 B1.

Accordingly, the surgical clip 10 of the first embodiment of invention is formed as a closed ring made (cut out) of an elastic material, for example a metal sheet or the like, and comprises a first elongated tissue grasping portion (surface) 12 having a first end 12 A and a second end 12B in the circumferential direction of the ring and a second elongated tissue grasping portion (surface) 14 also having a first end 14A and a second end 14B in the circumferential direction of the ring-shaped clip. As can be seen from the figures 2 to 4 both tissue grasping portions 12, 14 are located diametrical to each other and have a tissue grasping edge 13, 15, respectively, being formed in a straight or semi-circular elongation. Both tissue grasping edges 13, 15 are located at the respective tissue grasping portions 12, 14 such that they are orientated inside the ring-shaped surgical clip 10 to face each other.

A first joint or hinge portion 16 and a second joint or hinge portion 18 of the closed (integral) ring connect the first elongated tissue grasping portion 12 to the second elongated tissue grasping portion 14. For this purpose the first hinge portion 16 is connected at a first end 16A thereof to a first end 12A of the first elongated tissue grasping portion 12 and at a second end 16B thereof to a first end 14A of the second tissue grasping portion 14. Similarly, the second hinge portion 18 is connected at a first end 18A thereof to a second end 12B of the first elongated tissue grasping portion 12 and at a second end 18B thereof to a second end 14B of the second tissue grasping portion 14. Therefore, the above-mentioned closed ring is formed.

As can be further seen from figures 2 to 4 the rigidity of the tissue grasping portions 12, 14 is higher than the rigidity of the hinge portions 16, 18 at least in the ring plane, preferably by increasing the radial width of the material at the tissue grasping portions 12, 14 compared with the hinge portions 16, 18. Additionally, the ring is cambered in the rectangular direction with respect to the ring plane such that the tissue grasping portions 12, 14 get an arc-shape in a plane perpendicular to the ring plane.

In this embodiment, the first hinge portion 16 and the second hinge portion 18 include a semi-circular portion 16C, 18C, respectively (in the ring plane), which are disposed between the first and second end 16A, 16B as well as the first and second end 18A, 18B, thereof. These semi-circular portions 16C, 18C, however, extend toward the inside of the closed ring of the tissue clip 10, especially (because the clip 10 has a cambered shape as stated above) toward the first and second tissue grasping portions 12, 14.

By cambering the ring-shaped clip 10 in the area of the tissue grasping portions 12, 14, the clip 10 gets the shape of a shark mouth in which the both tissue grasping edges 13, 15 face each other in a closed position of the clip 10 (forming a narrow tissue grasping gap there between). In order to increase the grasping effect of the clip 10 both tissue grasping edges 13, 15 are formed with interlocking teeth 19 which extend from the tissue grasping portions 12, 14 inside the ring-shaped clip 10.

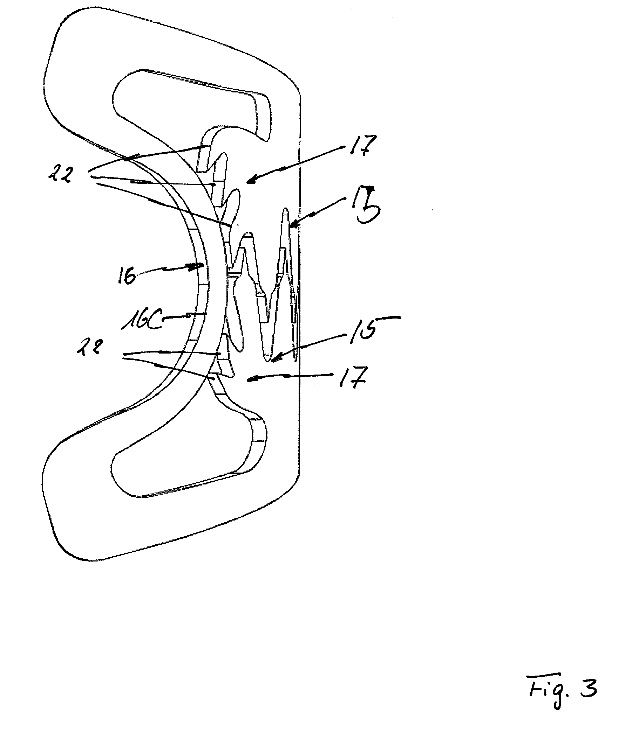

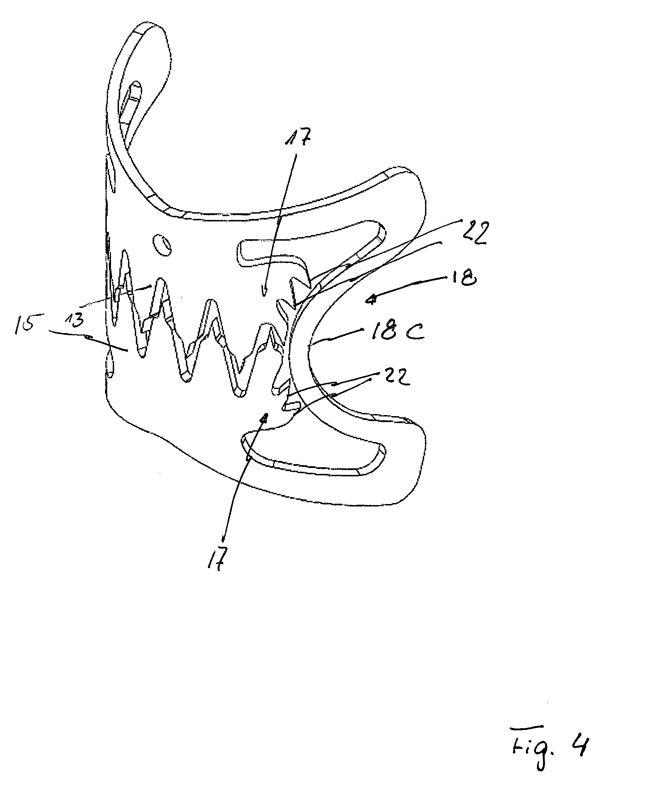

Especially, in accordance with figures 3 and 4, both tissue grasping portions 12, 14 comprise additional (auxiliary) grasping teeth 22, located at the respective ends 12A, 12B, 14A, 14B of each tissue grasping portion 12, 14. In other words, the first ends 12A, 14A as well as the second ends 12B, 14B of the first and second tissue grasping portions 12, 14 are formed with an additional edge 17 (in elongation with the tissue grasping edges 13, 15) facing the hinge portions 16, 18 such that a narrow gap is created there between. The additional edges 17 of the tissue grasping portions 12, 14 are formed with the additional grasping teeth 22 which extend to the inside of the ring and which are additionally inclined in the direction to the hinge portions 16 (their semi-circular portions 16C) to cross the narrow gap.

When grasping tissue between both tissue grasping portions 12, 14, the additional grasping teeth 22 will clamp the tissue also between the additional edges 17 of the tissue grasping portions 12, 14 and the hinge portions 16, 18 (semi-circular portions 16C) such that the overall grasping surface/grasping line is increased/extended compared with a tissue clip according to the prior art. Therefore, the grasping effect is enhanced without the necessity to increase the clamping force. For that reason, the risk of tissue damage created by the clip 10 can be reduced.

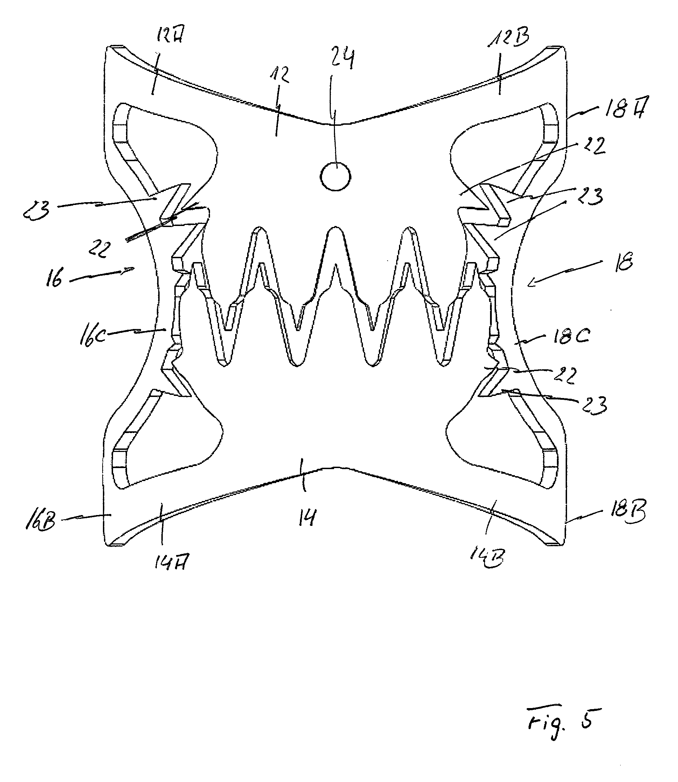

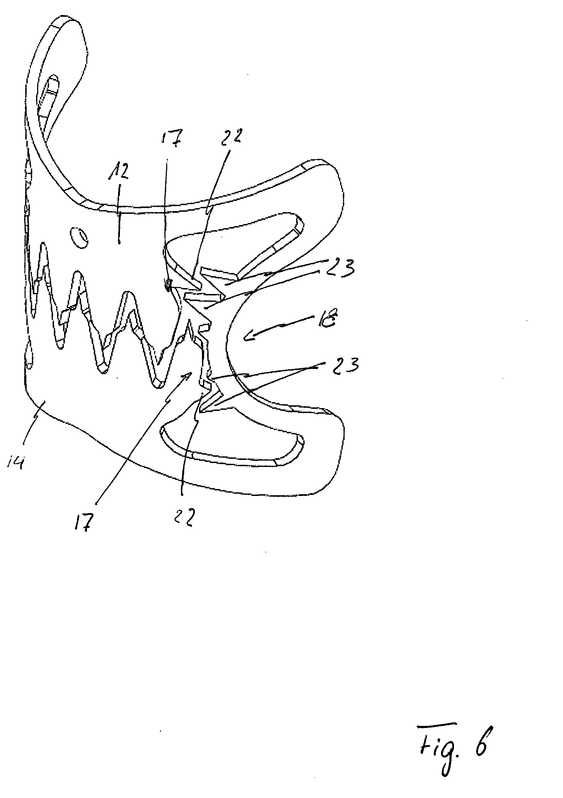

The figures 5 to 7 show a second preferred embodiment of the invention wherein in the following only those technical features will be described in more detail which are different to the first preferred embodiment of the invention. All non-described features are, therefore, identical with the first embodiment.

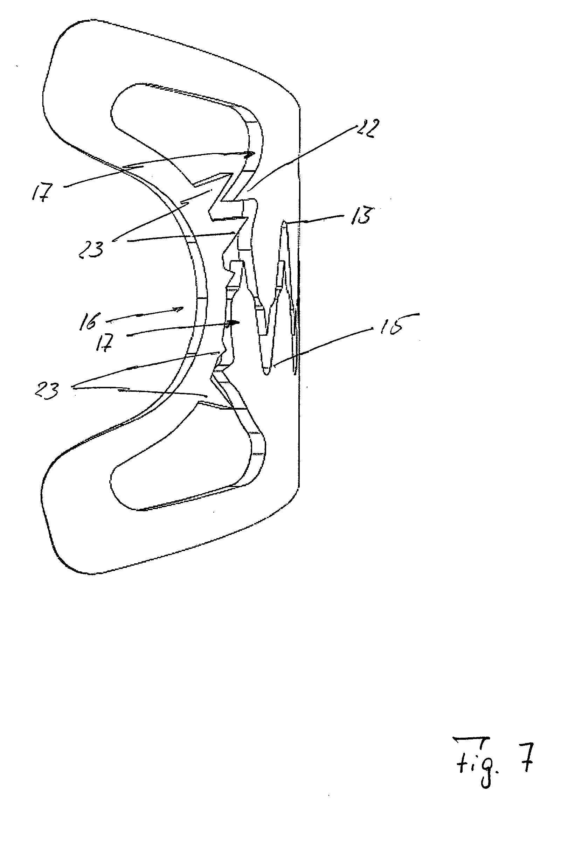

As can be best seen from figure 7, the hinge portions 16, 18 of the clip 10 according to the second embodiment of the invention, are formed with hinge teeth 23 which extend to the inside of the ring (in the ring plane) and are additionally inclined in the direction to the respective ends 12A, 12B, 14A, 14B or edges 17 of the opposite (facing) tissue grasping portions 12, 14 to co-operate with the additional grasping teeth 22 located there. Therefore, the grasping effect of the clip 10 along the hinge portions 16, 18 is increased compared with the first embodiment of the invention.

Here, it shall be clarified that a plurality of variations concerning the concrete design of the inventive clip 10 can be carried out by a skilled person making use of the inventive concept namely to incorporate also the hinge portions 16, 18 into the overall grasping area of the surgical clip 10. The most decisive issue of the invention, therefore, is to create an additional narrow gap between the hinge portions 16, 18 and the facing (opposite) ends/edges 17 of the tissue grasping portions 12, 14, respectively which gaps can be used to additionally clamp tissue there between. The arrangement of additional grasping teeth 22, 23 within the additional gaps at the ends/edges 17 of the regular tissue grasping portions 12, 14 and/or along the hinge portions 16, 18 is just an optional measure to increase the grasping effect of the clip 10. Therefore, only one tooth or a tooth row arranged at the ends/end edges 17 of the regular grasping portions 12, 14 and/or the facing hinge portions 16, 18 might be possible. Also just additional clamping edges without any teeth along the edges 17 and the hinge portions 16, 18 would be suitable to increase the grasping effect of the inventive clip 10.

As stated above, the inventive clip 10 can be used for closing cuts in body tissue but also to temporarily anchor/fix medical or surgical means inside the patient. Therefore, the inventive surgical clip 10 according to all embodiments of the invention comprises a through hole 24 at least at one of the grasping portions 12, 14 extending perpendicular to the ring plane. This through hole 24 serves as an eyelet exclusively for receiving a fixing means like a cord or string with which a medical instrument or the like can be fixed.

Finally, to summarise the gist of the invention, it refers to a surgical clip or anchor according to claim 1 or claim 2.

## Claims

1. Surgical clip comprising a closed ring integrally made from an elastic sheet material having a plurality of tissue grasping portions (12, 14) each formed with at least one tooth or a row of teeth (19), situated between two adjacent circumferential ends (17) of each tissue grasping portion (12, 14), extending substantially parallel with each other and to the inside of the ring and a plurality of elastically deformable hinge portions (16, 18) located between two neighbouring grasping portions (12, 14) in circumferential direction, said hinge portions (16, 18) have a curvature extending in the direction inside the ring, so that the circumference of each hinge portion and the circumferential end (17) of each adjacent grasping portion (12, 14) are at least partially facing each other forming a gap therebetween **characterised in that** at least one of said grasping portions (12, 14) is provided with an additional tooth or an additional row of teeth (22, 23) extending in the direction inside the ring and to the adjacent neighbouring hinge portions (16, 18), wherein said additional tooth or additional row of teeth (22, 23) is arranged in said gap creating additional grasping areas besides said grasping portions.

2. Surgical clip comprising a closed ring integrally made from an elastic sheet
material having a plurality of tissue grasping portions (12, 14) each formed with at least one tooth or a row of teeth (19), situated between two adjacent
circumferential ends (17) of each tissue grasping portion (12, 14), extending substantially parallel with each other and to the inside of the ring and a plurality of elastically deformable hinge portions (16, 18) located between two neighbouring grasping portions (12, 14) in circumferential direction, said hinge portions (16, 18) have a curvature extending in the direction inside the ring, so that the circumference of each hinge portion and the circumferential end (17) of each adjacent grasping portion (12, 14) are at least partially facing each other forming a gap
therebetween **characterised in that** at least one of, said hinge portions (16, 18) is provided with an additional tooth or an additional row of teeth (22, 23) extending in the direction inside the ring and to the adjacent grasping portions (12, 14), wherein said additional tooth or additional row of teeth (22, 23) is arranged in said gap creating additional
grasping areas besides said grasping portions.

3. Surgical clip according to claim 1 or 2, wherein said additional tooth or row of teeth (22, 23) is inclined with respect to the radius of the closed ring in the direction to the adjacent grasping portion (12, 14) and/or the adjacent hinge portion (16, 18) to get a circumferential direction component.

4. Surgical clip according to anyone of claims 1 to 3, wherein the clip (10) has two grasping portions (12, 14) located diametrical such that their tooth or row of teeth (19), except the additional tooth or additional row of teeth (22, 23), face each other.

5. Surgical clip according to claim 4, wherein said clip (10) is cambered in the ring plane such that said diametrically located grasping portions (12, 14) get a substantially arc-shaped curvature extending perpendicular to the ring plane.

6. Surgical clip according to anyone of claims 1 to 5, wherein said grasping portions (12, 14) have a radial sheet width larger than the sheet width of the hinge portions (16, 18) to increase their respective rigidity compared with the hinge portions (16, 18).

7. Surgical clip according to claim 6, wherein a through hole (24) formed in at least one of said grasping portions (12, 14) radial outside to said at least one tooth or row of teeth (19) and substantially halfway with respect to the circumferential extension of said at least one grasping portion (12, 14).

8. Surgical clip according to claim 7, wherein said through hole (24) extends perpendicular to the ring plane and forms a separate connection eyelet at which a medical device is anchorable or anchored.

9. Surgical clip according to claim 5, wherein, by cambering the ring-shaped clip (10) in the area of the tissue grasping portions (12, 14), said clip (10) gets the shape of a shark mouth in which the at least one tooth or row of teeth (19) of each tissue grasping portion (12,14) face each other in a closed position of said clip (10)

## Patentansprüche

1. Chirurgischer Clip, bestehend aus einem geschlossenen Ring, der aus einem
elastischen Blechmaterial stoffeinstückig gefertigt ist sowie eine Vielzahl von Gewebegreifabschnitten (12, 14) aufweist, von denen jeder mit wenigstens einem Zahn oder einer Zahnreihe (19) ausgebildet ist, die zwischen zwei umfangsseitigen Enden (17) jedes Gewebegreifabschnitts (12, 14) liegen und sich im Wesentlichen parallel zueinander und zur Innenseite des Rings erstrecken, und aus einer Vielzahl von elastisch verformbaren Scharnierabschnitten (16, 18), die in Umfangsrichtung zwischen zwei benachbarten Greifabschnitten (12, 14) angeordnet sind, wobei die Scharnierabschnitte (16, 18) eine sich zur Innenseite des Rings erstreckende Krümmung aufweisen, so dass der Umfang jedes Scharnierabschnitts und das umfangsseitige Ende (17) jedes benachbarten Greifabschnitts (12, 14) wenigstens teilweise einander zugewandt sind, wobei sie dazwischen einen Zwischenraum ausbilden, **dadurch gekennzeichnet, dass** wenigstens einer der Greifabschnitte (12, 14) mit einem zusätzlichen Zahn oder einer zusätzlichen Zahnreihe (22, 23) ausgebildet ist, die sich zur Innenseite des Rings und zu den benachbarten Scharnierabschnitten (16, 18) erstrecken, wobei der zusätzliche Zahn oder die zusätzliche Zahnreihe (22, 23) in dem Zwischenraum angeordnet sind und neben den Greifabschnitten einen zusätzlichen Greifbereich schaffen.

2. Chirurgischer Clip, bestehend aus einem geschlossenen Ring, der aus einem
elastischen stoffeinstückig Blechmaterial gefertigt ist sowie eine Vielzahl von Gewebegreifabschnitten (12, 14) aufweist, von denen jeder mit wenigstens einem Zahn oder einer Zahnreihe (19) ausgebildet ist, die zwischen zwei umfangsseitigen Enden (17) jedes Gewebegreifabschnitts (12, 14) liegen und sich im Wesentlichen parallel zueinander und zur Innenseite des Rings erstrecken, und aus einer Vielzahl von elastisch verformbaren Scharnierabschnitten (16, 18), die in Umfangsrichtung zwischen zwei benachbarten Greifabschnitten (12, 14) angeordnet sind, wobei die Scharnierabschnitte (16, 18) eine sich zur Innenseite des Rings erstreckende Krümmung aufweisen, so dass der Umfang jedes Scharnierabschnitts und das umfangsseitige Ende (17) jedes benachbarten Greifabschnitts (12, 14) wenigstens teilweise einander zugewandt sind, wobei sie dazwischen einen Zwischenraum ausbilden, **dadurch gekennzeichnet, dass** wenigstens einer der Scharnierabschnitte (16, 18) mit einem zusätzlichen Zahn oder einer zusätzlichen Zahnreihe (22, 23) ausgebildet ist, die sich zur Innenseite des Rings und zu den benachbarten Greifabschnitten (12, 14) erstrecken, wobei der zusätzliche Zahn oder die zusätzliche Zahnreihe (22, 23) in dem Zwischenraum angeordnet sind und neben den Greifabschnitten einen zusätzlichen Greifbereich schaffen.

3. Chirurgischer Clip nach Anspruch 1 oder 2, wobei der zusätzliche Zahn oder die
zusätzliche Zahnreihe (22, 23) bezüglich des Radius des geschlossenen Rings in Richtung des benachbarten Greifabschnitts (12, 14) und/oder des benachbarten Scharnierabschnitts (16, 18) geneigt sind, um eine umlaufende Komponente zu erhalten.

4. Chirurgischer Clip nach einem der Ansprüche 1 bis 3, wobei der Clip (10) zwei
Greifabschnitte (12, 14) aufweist, die diametral angeordnet sind, so dass deren Zahn oder Zahnreihe (19), ausgenommen des zusätzlichen Zahns oder der zusätzlichen Zahnreihe (22, 23), einander zugewandt sind.

5. Chirurgischer Clip nach Anspruch 4, wobei der Clip (10) in der Ringebene derart
gewölbt ist, dass die diametral angeordneten Greifabschnitte (12, 14) eine im Wesentlichen bogenförmige Krümmung annehmen, die sich senkrecht zur Ringebene erstreckt.

6. Chirurgischer Clip nach einem der Ansprüche 1 bis 5, wobei die Greifabschnitte (12, 14) eine radiale Blechbreite aufweisen, die größer ist als die Blechbreite der Scharnierabschnitte (16, 18), um deren Steifigkeit im Vergleich zu den Scharnierabschnitten (16, 18) entsprechend zu erhöhen.

7. Chirurgischer Clip nach Anspruch 6, wobei in wenigstens einem der
Greifabschnitte (12, 14) eine Durchgangsöffnung (24) radial außen zu dem wenigstens einen Zahn oder der Zahnreihe (19) und im Wesentlichen auf halber Strecke bezüglich der Umfangserstreckung des wenigstens einen Greifabschnitts (12, 14) ausgebildet ist.

8. Chirurgischer Clip nach Anspruch 7, wobei sich die Durchgangsöffnung (24)
senkrecht zu der Ringebene erstreckt und eine separate Verbindungsöse ausbildet, an der eine medizinische Vorrichtung verankerbar oder verankert ist.

9. Chirurgischer Clip nach Anspruch 5, wobei der ringförmige Clip (10) durch das
Wölben im Bereich der Gewebegreifabschnitte (12, 14) die Form eines Haifischmauls erhält, bei der sich wenigstens ein Zahn oder eine Zahnreihe (19) jedes Gewebegreifabschnitts (12, 14) in einer geschlossen Position des Clips (10) gegenüberliegen.

## Revendications

1. Clip chirurgical comprenant un anneau fermé réalisé d'un seul tenant à partir d'un matériau de feuille élastique ayant une pluralité de parties de préhension de tissu (12, 14) formées chacune d'au moins une dent ou d'une rangée de dents (19), située entre deux extrémités circonférentielles adjacentes (17) de chaque partie de préhension de tissus (12, 14), s'étendant de manière sensiblement parallèle les unes par rapport aux autres et à l'intérieur de l'anneau, et une pluralité de parties d'articulation (16, 18) élastiquement déformables situées entre deux parties de préhension voisines (12, 14) dans la direction circonférentielle, lesdites parties d'articulation (16, 18) présentant une courbure s'étendant dans la direction à l'intérieur de l'anneau, de sorte que la circonférence de chaque partie d'articulation et l'extrémité circonférentielle (17) de chaque partie de préhension adjacente (12, 14) se font au moins partiellement face en formant un espace entre elles, **caractérisé en ce qu'**une desdites parties de préhension (12, 14) est dotée d'une dent supplémentaire ou d'une rangée de dents supplémentaire (22, 23) s'étendant dans la direction à l'intérieur de l'anneau et vers les parties d'articulation voisines (16, 18), dans lequel ladite dent supplémentaire ou ladite rangée de dents supplémentaire (22, 23) est disposée dans ledit espace, créant des zones de préhension supplémentaires en plus desdites parties de préhension.

2. Clip chirurgical comprenant un anneau fermé réalisé d'un seul tenant à partir d'un matériau de feuille élastique ayant une pluralité de parties de préhension de tissu (12, 14) formées chacune d'au moins une dent ou d'une rangée de dents (19), située entre deux extrémités circonférentielles adjacentes (17) de chaque partie de préhension de tissu (12, 14), s'étendant de manière sensiblement parallèle les unes par rapport aux autres et à l'intérieur de l'anneau, et une pluralité de parties d'articulation (16, 18) élastiquement déformables situées entre deux parties de préhension voisines (12, 14) dans la direction circonférentielle, lesdites parties d'articulation (16, 18) présentant une courbure s'étendant dans la direction à l'intérieur de l'anneau, de sorte que la circonférence de chaque partie d'articulation et l'extrémité circonférentielle (17) de chaque partie de préhension adjacente (12, 14) se font au moins partiellement face en formant un espace entre elles, **caractérisé en ce qu'**au moins une desdites parties d'articulation (16, 18) est dotée d'une dent supplémentaire ou d'une rangée de dents supplémentaire (22, 23) s'étendant dans la direction à l'intérieur de l'anneau et vers les parties de préhension (12, 14) adjacentes, dans lequel ladite dent supplémentaire ou rangée de dents supplémentaire (22, 23) est disposée dans ledit espace, créant des zones de préhension supplémentaires en plus desdites parties de préhension.

3. Clip chirurgical selon la revendication 1 ou 2, dans lequel ladite dent ou rangée de dents supplémentaire (22, 23) est inclinée par rapport au rayon de l'anneau fermé en direction de la partie de préhension adjacente (12, 14) et/ou de la partie d'articulation adjacente (16, 18) pour adopter une composante de direction circonférentielle.

4. Clip chirurgical selon l'une quelconque des revendications 1 à 3, le clip (10) possédant deux parties de préhension (12, 14) situées de façon diamétrale de telle sorte que leur dent ou rangée de dents (19), excepté la dent supplémentaire ou rangée de dents supplémentaire (22, 23), se font face.

5. Clip chirurgical selon la revendication 4, dans lequel ledit clip (10) est cambré dans le plan de l'anneau de telle sorte que lesdites parties de préhension situées de façon diamétrale (12, 14) adoptent une courbure sensiblement arquée s'étendant perpendiculairement au plan de l'anneau.

6. Clip chirurgical selon l'une quelconque des revendications 1 à 5, dans lequel lesdites parties de préhension (12, 14) ont une largeur de feuille radiale plus importante que la largeur de feuille des parties d'articulation (16, 18) pour augmenter leur rigidité respective par rapport aux parties d'articulation (16, 18).

7. Clip chirurgical selon la revendication 6, dans lequel un trou traversant (24) est formé dans au moins l'une desdites parties de préhension (12, 14) radialement depuis l'extérieur vers ladite au moins une dent ou rangée de dents (19) et sensiblement à mi-chemin par rapport à l'extension circonférentielle de ladite au moins une partie de préhension (12, 14).

8. Clip chirurgical selon la revendication 7, dans lequel ledit trou traversant (24) s'étend perpendiculairement au plan de l'anneau et forme un oeillet de liaison séparé auquel un dispositif médical peut être ancré ou est ancré.

9. Clip chirurgical selon la revendication 5, dans lequel, en cambrant le clip de forme annulaire (10) dans la zone des parties de préhension de tissu (12, 14), ledit clip (10) adopte la forme d'une gueule de requin dans laquelle l'au moins une dent ou rangée de dents (19) de chaque partie de préhension de tissu (12, 14) se font face dans une position fermée dudit clip (10).
